# EUROPEAN PATENT APPLICATION

(11) **EP 1 733 631 A1**
(43) Date of publication of application: **20.12.2006**
(21) Application number: 05710676.7
(22) Date of filing: 18.02.2005
(51) Int. Cl.: A23L 1/30, A23L 1/305, A23L 1/325, A61K 35/60, A61P 25/00, A61P 25/18, A61P 43/00

(54) **FOOD COMPOSITION HAVING ANTISTRESS EFFECT**

(30) Priority: 12.03.2004 JP 2004070109
(71) Applicant: Ajinomoto Co., Inc., Tokyo 104-8315 (JP)
(72) Inventor: Ishizaki, Taichi, c/o Ajinomoto Co., Inc., Kawasaki-shi, Kanagawa 2108681 (JP); Kuroda, Motonaka, c/o Ajinomoto Co., Inc., Kawasaki-shi, Kanagawa 2108681 (JP); Honda, Masashi, c/o Ajinomoto Co., Inc., Kawasaki-shi, Kanagawa 2108681 (JP); Yamada, Keiko, c/o Ajinomoto Co., Inc., Kawasaki-shi, Kanagawa 2108681 (JP)
(74) Representative: Strehl Schübel-Hopf & Partner
(86) International application number: PCT/JP2005/003075
(87) International publication number: WO 2005/087022

(57) **Abstract**

In this application is disclosed a food composition having an anti-stress action, which composition comprises, as an active ingredient, an extract of migratory fish or a mixture of nutritional components of a similar composition to the above-mentioned extract in an amount of 0.5 g or more per one meal of the extract or the mixture in terms of the solid content thereof. With the food composition it is possible to prevent or alleviate states such as fatigue e.g., asthenopia, mental fatigue, emotional dysfunction and the like, caused by stress and felt on a daily basis.

## Description

### TECHNICAL FIELD

The present invention relates to a food composition having an anti-stress action, which composition comprises, as an active ingredient, an extract of migratory fish or a mixture of nutritional components in a similar composition to the extract, said composition being effective for those who feel psychological stress such as lethargy, mental fatigue, emotional dysfunction, an unstable emotional state or the like, on a daily basis.

### BACKGROUND ART

In recent years, the relation of stress to the causes of various illnesses and disorders has been suggested. Stress also gives rise to mental fatigue, emotional dysfunction and an unstable emotional state that are felt on a daily basis. A maj or problem in this situation is that the social environmental conditions surrounding people in daily life are forming an environment that further increases stress. Against this background, there is a strong demand for the development of a drug or a food or drink having an anti-stress action that can effectively and safely prevent or alleviate various disorders caused by stress.

Examples of conventional anti-stress agents include pharmaceuticals and the like, containing tranquilizers or the like. However, these pharmaceuticals are not suitable for many people who feel mental fatigue or emotional dysfunction on a daily basis, as the effect of the pharmaceuticals is too strong.

As foods having an anti-stress action, there have been reported a food composition which contains cacao bean extract (see, Japanese Patent Application Laid-Open (Kokai) No. 9-206026), a food composition containing sweet potato extract (see, Japanese Patent Application Laid-Open (Kokai) No. 2001-335505), a composition containing extract of the bark of a pine tree (see, Japanese Patent Application Laid-Open (Kokai) No. 2003-95964), a food containing zedoary or zedoary extract (see, Japanese Patent Application Laid-Open (Kokai) No. 2003-38125), and the like. However, the mechanism of how these foods act has not been completely clarified, and the effect of these foods can not be adequately felt inhumans either. Further, these reported foods are generally provided in a pharmaceutical form, and it is difficult to utilize them by mixing with other existing foods because of their sensory properties, physicochemical properties and the like. When the purpose is to ingest such type of foods on a routine basis as part of one's daily diet to prevent or alleviate stress, it is considered that, where possible, the inventive foods should preferably not only be in the form of a supplement but also be capable of being mixed in general foods to provide a delicious taste, and that the foods that have undergone a widespread ingestion experience are more preferable. From these viewpoints, since the components of the above-mentioned known foods, pharmaceuticals and supplements have limited application to foods, there has been a demand for the development of a food that has versatility, and plentiful experience of ingestion, and is more effective.

On the other hand, it is known that anserine and carnosine, which are contained in seafood and animal meat in large quantities, activate ATPase. Japanese Patent Application Laid-Open (Kokai) No. 2002-173422 discloses that enhancement of exercise capacity and anti-fatigue effect are exhibited by administrating at least one kind selected from imidazole peptides, especially anserine, carnosine, balenine and the like, obtained by purifying specifically low molecular fractions of extracts of seafood, chicken meat and animal meat by allowing them to pass through an ultrafilter membrane. However, this publication does not discuss the effect of the composition of the present invention for a stress condition such as mental fatigue or the like.

Further, in Japanese Patent Application Laid-Open (Kokai) No. 9-20661, it is shown that a composition comprising one or more kinds of imidazole compound selected from the group consisting of anserine, carnosine, balenine, π-methylhistidine and τ-methylhistidine alleviates lethargy in human caused by excessive mental activity and has an anti-mental fatigue effect that increases concentration.

Meanwhile, Japanese Patent Application Laid-Open (Kokai) No. 9-20660 discloses an anti-stress food or drink produced by adding one or more kinds of imidazole compound selected from the group consisting of anserine, balenine, π-methylhistidine and τ-methylhistidine, or an extract having this as the main ingredient. In the examples therein, calculating ability tests as well as short term memory tests were conducted for human subjects. In this patent document, however, the term "an extract having an imidazole compound as the main ingredient" refers to, for example, a fraction isolated from liquid concentrate obtained from fish processing using ultrafiltration and ion exchange resin (see, Example 6 (Paragraph 0039) in the same document), and the content of an imidazole compound therein is 2% or more, preferably 10% or more, and more preferably 50% or more, and in particular the content of anserine is 1% or more, preferably 5% or more, and more preferably 20% or more (see, Paragraphs 0014-0015 in the same document). It is considered that the cost of producing this composition is high, and that the composition is thus unrealistic as a common food.

### DISCLOSURE OF THE INVENTION

### [Problems to be Solved by the Invention]

In consideration of the background art described in the previous section, it is an object of the present invention to provide a food composition which prevents, improves or alleviates lethargy, mental fatigue, mental dysfunction and a state of mental instability that are felt on a daily basis and can be taken in the form that allows easy ingestion in normal dietary life, and which food composition has an anti-stress action, which in turn has an anti-fatigue action for a mental load, and furthermore an action that improves the efficiency of mental labor as a result of these actions.

### [Means for Solving the Problems]

The present inventors have conducted concentrated studies to achieve the aforementioned object, and found that an extract of migratory fish such as bonito or the like, or an extract of dried and smoked fish made from migratory fish such as bonito or the like has an action that alleviates lethargy such as asthenopia or the like, and stress such as mental fatigue, mental dysfunction or the like, that are felt on a daily basis, and also improves the efficiency of mental labor, and have completed the present invention on the basis of these findings.

Accordingly, the present invention relates to a food composition having an anti-stress action, which composition comprises, as an active ingredient, an extract of migratory fish such as bonito or the like, or an extract of dried fish made from migratory fish such as bonito or the like, and also to a food composition having an anti-stress action, comprising, as an effective ingredient, a mixture of nutritional components of a similar composition to the above-mentioned extracts, said mixture including (a) a total of 294 mg or more of nitrogen-containing compounds such as peptides, proteins, amino acids, and the like, derivable from a bonito extract, (b) a total of 55 mg or more of organic acids consisting of lactic acid, citric acid, malic acid and succinic acid; (c) 1 mg or more of nucleic acid-related compounds; and (d) a total of 16 mg or more of mineral compounds such as NaCl and KC1, at a ratio by weight of, when the amino acid amount is defined as 1, from 0.5 to 2 of the organic acid, from 0.001 to 0. 5 of the nucleic acid-related compound and from 0.02 to 2 of the mineral component. Further, the present invention related to a food composition prepared in such that the food composition contains 0.5 g or more per one meal of such extract in terms of the solid content thereof, whereby such above-mentioned food compositions may be easily ingested in an effective amount (0.5 g or more per one meal in terms of the solid content of such extract) on a daily basis. Examples thereof include a liquid food composition prepared in such that a liquid extract of migratory fish is aseptically packaged to contain 0.5 g or more per one meal of an extract of migratory fish in terms of the solid content thereof, and such that the dissolved oxygen concentration in the extract liquid is 5 ppm or less, a solid food composition such as a powdered, granulated, capsulated or tabletted food composition prepared in such that such extract is added with an excipient and a taste-correcting component, followed by powdering, granulating, capsulating or tableting, to contain 0.5 g or more per one meal of such extract in terms of the solid content thereof, and a jellified food composition prepared in such that such an extract of migratory fish is added with at least one kind of gelling agent selected from the group consisting of agar, gelatin, starches and gums, to contain 0.5 g or more per one meal of the extract of migratory fish in terms of the solid content thereof.

### BRIEF DESCRIPTION OF DRAWINGS

Fig. 1 shows the changes in the emotion and mood states by the POMS test (Test Example 2).
Fig. 2 shows the change in the total mood disturbance (TMD) (Test Example 2).
Fig. 3 shows the changes in the mood before and after the ingestion of an extract of dried bonito (Test Example 2).
Fig. 4 shows the amounts of corticosterone after restraint stress (Test Example 5).
Fig. 5 shows the LDH activity after restraint stress (Test Example 5).
Fig. 6 shows the GOT activity after restraint stress (Test Example 5).

### BEST MODE FOR CARRYING OUT THE INVENTION

Hereinafter, the invention will be explained in greater detail.

The term "migratory fish" as used in connection with the present invention refers to fish that carry out a large migration on a regular basis for the purpose of egg production or according to the season, including bonito, tuna, swordfish, mackerel, sardine, salmon, herring, amberjack and the like. Use of fish belonging to Perciformes Scombrina such as bonito, mackerel, bullet mackerel, butter fish, southern bluefin tuna, tuna, bluefin tuna, albacore, bigeye tuna and swordfish is especially preferable in view of the positive effect thereof. Here, this classification is based on "Genshoku Gyorui Kensaku Zukan (Primarily Colored Picture Book for Searching Fish) (revised 13^{th} edition)" (published in 1989 by Hokuryukan).

A method for producing the extract of the migratory fish for use in the present invention is not particularly limited, and the extract may be obtained by an ordinary method, or an extract produced by a method different from an ordinary method may be used as long as it has a similar composition.

More specifically, the extract of a migratory fish usable in the invention includes an extract obtained, for example, by concentrating broth and/or steam-cooked broth of fish meat such as bonito or the like to around Brix 20 to 40 according to an ordinary method and filtrating, degrading enzymatically the filtrated liquid with protease at 20 to 60 °C for a period within a range of 1 minute to 24 hours, concentrating the product to around Brix 40 to 60, adding sugar (such as glucose, fructose and the like) within a range of 0.1 to 10 % and, subsequently, subjecting the resulting product to heating and browning processing at 60 to 150 °C for a period within a range of 1 minute to 24 hours. As a further example, there may be mentioned a filtrate that has been obtained by heating and concentrating broth and/or steam-cooked broth of bonito to around Brix 20 to 40 according to an ordinary method, followed by filtrating the resulting concentrate after added and mixed with diatomaceous earth in an amount of 2 - 20 % based on the resulting concentrate.

Further, an extract obtained from dried fish obtained, in turn, from migratory fish such as bonito, tuna, mackerel or the like, can also be used for the purpose of the present invention. More specifically, dried fish (ara-bushi in Japanese) obtained by cutting raw migratory fish, boiling and then roast-drying (baikan in Japanese), and a dried and molded fish product (kare-bushi in Japanese) obtained by molding dried fish (ara-bushi in Japanese), and the like, can all be used as the sourse of such extract for the purpose of the present invention. I.e., after obtaining a liquid extract from the dried fish as raw material by conducting hot-water extraction, such an extract is obtained from the liquid extract in that state or by concentrating and drying. Further, it is also possible to obtain such an extract by adding water to the aforementioned dried fish as raw material, followed by adding thereto a proteolytic enzyme such as protease, peptidase or the like to carry out enzyme degradation, and then conducting hot-water extraction.

This kind of extract may also be used in a state where its salt has been removed by electrodialysis, a reverse osmosis filtration method, or the like. Further, in order to make such an extract obtained from migratory fish or dried migratory fish easy to eat or drink, it is also possible to use such an extract after performing steam distillation treatment under reduced pressure or under normal pressure, deodorization or decolorization treatment using activated carbon or diatomaceous earth filtration, or the like. Such an extract can also be obtained by treatment with an ultrafiltration membrane with a nominal molecular cutoff of 500 to 5,000, preferably 500 to 4,000, and more preferably 1,000 to 2,000, or a reverse osmosis membrane with a nominal salt rejection of 10% or less, and preferably 5% or less for the purpose of deodorization or decolorization.

A composition having a composition similar to that of a bonito extract can also be employed in place of the bonito extract. As such composition, there may be mentioned a composition containing, as an effective ingredient, a mixture of nutritional components such as (a) 294 mg or more in total of nitrogen-containing compounds such aspeptides,proteins,amino acids, and the like, derivable from an extract of bonito, (b) 55 mg or more in total of organic acids consisting of lactic acid, citric acid, malic acid and succinic acid, (c) 1 mg or more in total of nucleic acid-related compounds and (d) 16 mg or more in total of mineral components such as NaCl and KCl, at weight ratios of, when defining the amount of the amino acids as 1, from 0.5 to 2 of the organic acids, from 0.001 to 0.5 of the nucleic acid-related compounds and from 0.02 to 2 of the mineral components. In this connection, the term "nucleic acid-related compounds" in such composition refers to nucleic acids, nucleotides, nucleosides and nucleic acid bases.

In addition to an extract of a migratory fish or a mixture of nutritional components having a composition similar to that of the extract, the food composition having an anti-stress action of the present invention may be mix with suitable additives in amounts that do not prohibit the effect of the present invention.

Examples of such additive include other nutritional components that promote the fatigue recovery effect of the migratory fish extract such as carbohydrates (glucose, sucrose, starch, and the like), lipids (vegetable oil, fish oil, animal fat, and the like), proteins (soybean protein, milk protein, and the like), minerals (inorganic salts such as potassium salt, sodium salt, calcium salt, and the like), vitamins (thiamine, niacin, vitamin C, carotene, and the like); anti-fatigue components such as taurine, caffeine, and the like; corrigent components such as sucrose, aspartame, acesulfame K, sodium glutamate, sodium chloride, and the like, suitable for imparting functions as a food (taste, eating feeling, safety, and the like) ; excipients such as carbohydrates, inorganic salts, and the like for granulation, comminution or crushing or solidification for the similar purpose of rendering the composition easy to eat; bacteriostatic components such as ethyl alcohol, acetic acid, sodium acetate, glycine, and the like, for a similar purpose; and pigments such as natural pigments, e.g., caramel pigment, annatto pigment, safflower pigment, paprika pigment, monascus pigment, grape pigment, and the like, and various synthetic pigments for a similar purpose. In order to provide the extract in a food form that is easier to eat, the food can also be provided after being mixed with, for a liquid food, a seasoning material such as soy sauce, miso soybean paste, a sweet rice wine for cooking, sugar, various extracts and the like, and for a powdered food, a seasoning material such as powdered soy sauce, powdered miso soy bean paste, powdered sake liquor, sugar, sodium chloride, various extract powders and the like. An additive or a food raw material for use herein is not limited to the foregoing, and naturally, other additives or food raw materials that are normally used in this field can also be mixed.

In order to make a food composition that contains an effective amount (weight amount of the extract in terms of the solid content thereof being 0.5 g or more, preferably 1.0 to 10 g per one meal) of the extract obtained by the above-described method and that is in a form that is easy to ingest, the following processing can be conducted, in addition to mixing with such corrigent components, other nutritional components and the like, as described above. I.e., when the inventive extract is to be provided in the form of a liquid food composition, a desired liquid food composition can be obtained by aseptically packaging a solution of the extract in such that the weight of the extract in terms of the solid content thereof is 0.5 g or more and that the dissolved oxygen concentration in the solution is 10 ppm or less (25 °C). A container to be used for such packing is preferably an aluminum pouch, an aluminum can, a steel can, a paper pack with inner walls that have been subjected to deoxygenation treatment, or a container composed of a packaging material that has a deoxygenating property or a packaging material that has an oxygen barrier property. For a liquid food composition, although the mixing of the composition with sodium chloride, alcohol or a food raw material containing these enables the provision of a composition that allows normal temperature marketing, in order to allow the extract to be ingested more effectively, an extract solution with a low salt content is preferably packaged aseptically after sterilization. In this connection, since deterioration in the flavor of the extract due to oxidation caused by the dissolved oxygen is then a problem, such packaging must be carried out in such that the dissolved oxygen concentration at 25 °C is 5 ppm or less, preferably 3 ppm or less, and more preferably 2 ppmor less. When the inventive extract is to be provided in the form of a solid food composition, excipients and corrigent, or taste-improving , components may be added thereto in such that the extract will be in an amount of 0.5 g or more in terms of the solid content thereof per one meal, i.e., such solid food composition will contain the inventive extract in an amount of 0.5 g or more in terms of the solid content thereof per one meal, followed by powdering, granulation or tabletting to obtain the desired solid food composition. Examples of excipients to be used at this time include carbohydrates such as glucose, sucrose, trehalose, maltose, lactose, dextrin, starch, and the like, peptides and proteins such as gelatin, casein and a partial hydrolysate thereof. Further, to impart functions as a food (taste, mouthfeel and the like), a suitable corrigent component such as sucrose, aspartame, acesulfame K, sodium glutamate, sodium chloride, or the like is preferably mixed. As a method for powdering, there may be mentioned spray drying, lyophilization, drum drying under vacuum or drum drying under normal pressure, after concentrating the extract if necessary. Regarding the extract, to allow the extract to be ingested without the taste being affected, the extract can be provided by packing it in a capsule after being powdered by the foregoing method. Further, as a means for providing the extract in a state in which it is easy to eat, a component having a gelling action can be added thereto to provide the extract as a jelly food. As the gelling agent to be used at this time, one or more kinds of agar, gelatin, starches and gums may be used. In this case also, to make the food more easily ingestible, the aforementioned corrigent components such as sucrose, aspartame, acesulfame K, sodium glutamate, sodium chloride, and the like, are preferably mixed therein.

The food composition of the present invention produced in the above-described manner can be placed in distribution as it is, more specifically, in the form of a liquid mixture, a powder mixture or the like as appropriate.

Finally, the dosage (ingestion amount) of the food composition of the present invention will be described. The results of studies where a bonito extract was administered to mice showed that an anti-stress action was observed when the dosage was of an amount exceeding 630 mg/kg in terms of the dried matter. When this dosage is converted from animals to human, it is 0.5 g or more in terms of the dried matter per one meal based on 3 meals per day for 1 human adult. When a dosage is of less than this amount, an effect cannot be expected. Regarding the upper limit of the ingestion amount, for extracts of migratory fish such as bonito and the like, although there is no particular limitation since they have an abundant ingestion experience and ingestion history, it is considered that an adequate effect can be obtained with an ingestion amount of 10 g per one meal. Further, although the principal targets of the food composition of the present invention are people who feel stress on a daily basis, an effect can also be obtained when the food composition is administered to people suffering from mental disorders or depression.

### [Examples]

The present invention will be described in further detail hereunder by means of examples and test examples.

### Test Example 1: Preparation of bonito extract and dried-bonito extract

Bonito extract was prepared by the following method. That is, a bonito extract was prepared by heating a broth of bonito meat to concentrate to around Brix 30 according to an ordinary method, then adding diatomaceous earth (Radiolite #700, manufactured by Showa Chemical Industry Co., Ltd.) to a content of 5.0% relative to the solid content of the extract and mixing, followed by filtering the resulting mixture using a filter cloth and desalting using an electrodialyzer (Acilyzer, manufactured by Asahi Kasei Corporation). The thus prepared extract will be hereinafter referred to as "Bonito Extract A". Another bonito extract was prepared by heating a broth of bonito meat to concentrate to around Brix 30 according to an ordinary method, followed by filtrating the concentrate, subjecting the filtrate to enzymatic hydrolysis with a protease at 50 °C for 3 hours, followed by deactivating the enzyme by heating at 90 °C for 10 minutes, concentrating the resulting mass to around Brix 60, adding sugar (glucose) in an amount of 2% to the concentrate, followed by subjecting the mixture to browning treatment by heating at 90 °C for 1 hour, and finally subjecting the browned mass to electrodialysis, as described above. The thus prepared extract will be hereinafter referred to as "Bonito Extract B" . Further, ultrafiltration was conducted for the aforementioned Bonito Extract A using an ultrafiltration membrane (manufactured by Millipore; Prep/Scale TFF6) with a nominal molecular cutoff of 1,000 to obtain a permeated liquid. This permeated liquid will be hereinafter referred to "as Bonito Extract C". In addition, reverse osmosis membrane treatment was conducted for the aforementioned Bonito Extract A using a reverse osmosis membrane with a nominal salt rejection of 5% (manufactured by Nitto Denko Corporation; NTR-7410HG) to obtain a permeated liquid of almost the same composition as Bonito Extract C.

Further, a dried-bonito extract was prepared by the following method. That is, after crushing 250 kg of dried bonito (ara-hombushi in Japanese) that had been prepared by an ordinary method, the crushed mass was immersed in 1, 000 kg of ion exchanged water that had been heated to 95 °C, and extraction was conducted at 90 °C for 45 minutes, followed by cloth filtering and membrane filtering to obtain an liquid extract (dry matter 5.0 %) . After subjecting this liquid extract to a nitrogen purge and then sterilizing by heating at 130 °Cfor 60 seconds, the liquid extract was aseptically packaged in an aluminum pouch. The thus prepared liquid extract will be hereinafter referred to as "Dried-Bonito Extract A". The dissolved oxygen concentration at this time was 1.0 ppm. Further, after crushing 250 kg of a rough dried bonito (ara-hombushi in Japanese) that had been prepared by an ordinary method, the crushed mass was immersed in 1,000 kg of ion exchanged water that had been heated to 50 °C, a protease was added thereto, and enzymatic hydrolysis was then conducted for 1 hour. Thereafter, hot-water extraction was conducted at 90 °C for 45 minutes, followed by cloth filtering and filter filtering to obtain a liquid extract (solid content 7.0%). After subjecting this extract to a nitrogen purge and then sterilizing by heating at.130 °C for 60 seconds, the liquid extract was aseptically packaged in an aluminum pouch. The thus prepared liquid extract will be hereinafter referred to as "Dried-Bonito Extract B". The dissolved oxygen concentration at this time was 1.1 ppm.

The component analysis values per solid content of these bonito extracts and dried-bonito extracts are shown in Table 1. Since each extract showed a similar component composition, as shown in the table, it was predicted that a similar effect could be expected for each extract.

**Table 1: Component Analysis Values**

| Analysis Items | | Bonito Extract A | Bonito Extract B | Bonito Extract C | Dried Bonito Extract A | Dried Bonito Extract B | Unit |
|---|---|---|---|---|---|---|---|
| Minerals | | 1.99 | 0.59 | 2.13 | 6.98 | 7.00 | g/100g |
| Total nitrogen content | | 13.6 | 14.4 | 11.6 | 13.0 | 15.0 | %(g/dL) |
| Organic acids Organic | Lactic acid | 11.70 | 9.20 | 16.10 | 17.50 | 8.57 | g/100g |
| | Acetic acid | 0.00 | 0.00 | 0.00 | 0.00 | 1.14 | g/100g |
| | Pyroglutamic acid | 0.41 | 0.67 | 0.70 | 0.00 | 0.00 | g/100g |
| Sugar composition | Maltose | 0.24 | 0.00 | 0.33 | 0.00 | 0.00 | g/100g |
| | Fructose | 0.10 | 0.01 | 0.14 | 0.00 | 0.00 | g/100g |
| | Xylose | 0.00 | 0.00 | 0.00 | 0.00 | 0.14 | g/100g |
| | Glucose | 0.86 | 0.15 | 1.22 | 0.00 | 0.57 | g/100g |
| Free amino acids | P-Ser | 175.7 | 278.1 | 258.8 | 82.8 | 121.8 | mg/100g |
| | Tau | 2567.7 | 2148.6 | 4003.3 | 1194.6 | 1721.8 | mg/100g |
| | Asp | 312.1 | 357.0 | 495.7 | 130.1 | 164.3 | mg/100g |
| | Thr | 208.6 | 233.7 | 341.6 | 98.5 | 277.5 | mg/100g |
| | Ser | 255.2 | 283.3 | 400.4 | 128.5 | 231.8 | mg/100g |
| | Glu | 447.7 | 265.3 | 756.0 | 129.3 | 548.2 | mg/100g |
| | Gly | 273.6 | 296.9 | 417.9 | 177.3 | 151.4 | mg/100g |
| | Ala | 746.2 | 807.1 | 1124.0 | 584.9 | 736.1 | mg/100g |
| | Val | 328.4 | 659.6 | 617.4 | 248.4 | 297.1 | mg/100g |
| | Met | 210.2 | 443.4 | 398.4 | 246.2 | 451.4 | mg/100g |
| | Ile | 243.1 | 315.6 | 421.9 | 110.3 | 332.9 | mg/100g |
| | Leu | 577.7 | 662.1 | 943.5 | 284.5 | 1222.5 | mg/100g |
| | Tyr | 285.7 | 297.6 | 435.3 | 133.2 | 397.9 | mg/100g |
| | Phe | 301.7 | 330.7 | 524.1 | 87.7 | 552.6 | mg/100g |
| | NH3 | 109.1 | 113.5 | 133.6 | 987.1 | 469.3 | mg/100g |
| | Lys | 741.4 | 718.5 | 1105.1 | 547.0 | 1803.6 | mg/100g |
| | His | 10552.6 | 7692.6 | 14957.6 | 13662.1 | 7455.7 | mg/100g |
| | Ans | 1123.4 | 1052.1 | 1521.4 | 1177.4 | 1251.1 | mg/100g |
| | Car | 343.4 | 321.7 | 439.3 | 373.7 | 335.7 | mg/100g |
| | Arg | 371.5 | 429.1 | 458.3 | 144.6 | 1149.5 | mg/100g |
| | Pro | 483.1 | 253.3 | 541.1 | 393.5 | 170.5 | mg/100g |

### Test Example 2: Confirmation of action for mental state of human

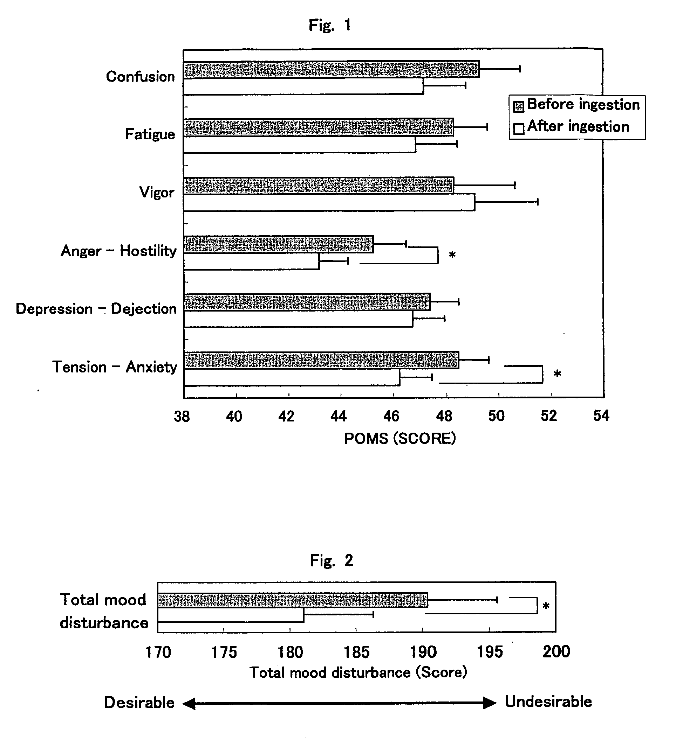

A solution (liquid food composition) of the Dried-Bonito Extract A of the present invention that was prepared in Test Example 1 was provided to a group of 17 (n=17) healthy female subjects between 20 and 29 years old for ingestion for 7 days at morning and evening meals in amounts of 50 mL at each meal, representing a daily ingestion amount of 100 mL. Before and after the ingestion period, the subjects underwent a POMS test (Profile of Mood States; Kaneko Shobo Co., Tokyo, No.850) (Yokoyama et al., Nihon Koushyuu Eisei Zasshi, Vol. 37, 913-918, 1990 (an article in Japanese)) and answered a mood questionnaire concerning fatigue and the like (the method according to Kikuchi et al. modified. Shinyaku to Rinshou, Vol. 51, 525-530 (2002) (an article in Japanese)), to confirm the influence of ingestion of the dried-bonito extract on the changes in mood and emotion. At this time, a solution obtained by mixing a dried-bonito flavor extract, a caramel pigment and sodium chloride was used as a placebo sample. The test was conducted by means of a single-blind, crossover study. The results of the POMS tests before and after ingestion of the dried-bonito extract will be shown in the accompanying Figs. 1 and 2. In the figures, items that showed a significant difference at p<0.05 or less in comparison to before ingestion are represented by a * symbol.

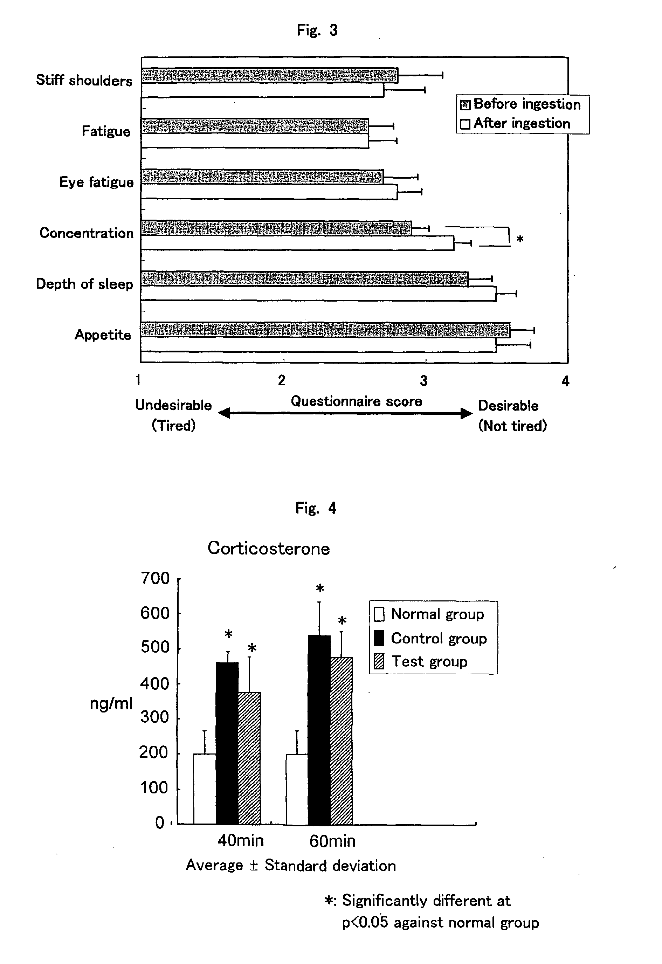

As shown in Figure 1, for the subjects who ingested the aforementioned dried-bonito extract, scores for the items "tension - anxiety" and "anger - hostility" after ingestion were significantly lower than those before ingestion. Further, TMD (a value obtained by subtracting the value for "vigor" from the total of the scores of the 5 items other than "vigor"), an index that shows total mood disturbance, was also decreased significantly (Figure 2). Although a significant difference was not observed in the scores for "depression - dejection", "confusion" and "fatigue", a trend was seen that showed a lower value after ingestion of the dried-bonito extract in comparison to the value before ingestion. Although a significant difference was not observed for the scores for "vigor", a trend was seen that showed a higher value after ingestion of the dried-bonito extract in comparison to the value before ingestion. These results showed that the mental state is improved by one week of continued ingestion of the dried-bonito extract. Further, as shown in Figure 3, it was shown that the score for "concentration" in the mood questionnaire was significantly increased as a result of ingestion of the dried-bonito extract. Also for the items "eye fatigue" and "depth of sleep", although a significant difference was not observed, a trend was seen that showed a higher value after ingestion (an improved state) . Based on these results, it was shown that ingestion of dried-bonito extract improved mental state and alleviated stress for healthy subjects.

### Test Example 3: Confirmation of action for anti-fatigue, stress marker and calculation ability at time of calculation load

A solution (liquid food composition) of Dried-Bonito Extract A of the present invention that was prepared in Test Example 1 was provided to a group of 31 (n=31) healthy female subjects between 20 and 29 years old for ingestion for 7 days at morning and evening meals in amounts of 62.5 mL at each meal (125 mL per day). Before and after the ingestion period, the subjects underwent Uchida-Kraepelin's (calculation) test (prepared by Nisseiken Inc. (Japan Mental Technology Institute Inc.)), and flicker values were measured prior to (pre-load) and after (post-load) this test, and measurement of salivary cortisol was also conducted prior to the test (pre-load) . In this connection, for the purpose of familiarizing the test subjects with the aforementioned Uchida-Kraepelin's test, the same type of test was also conducted one week prior to the start of ingestion. Table 2 shows the results of measurement of the flicker values before and after ingestion of the solution of the dried-bonito extract (values before and after the calculation test as well as the difference between the two values) . Further, the values for salivary cortisol before and after ingestion of the same solution are shown in Table 3, and the results for rate of errors in Uchida-Kraepelin' s test before and after ingestion of the same solution are shown in Table 4. In the tables, the values that showed a significant difference at p<0.05 in comparison to before ingestion are marked with a * symbol, and those that showed a significant difference at p<0.01 in comparison to before ingestion are marked with a ** symbol. Further, the values that showed a significant difference at p<0.05 between before and after the load are marked with a # symbol.

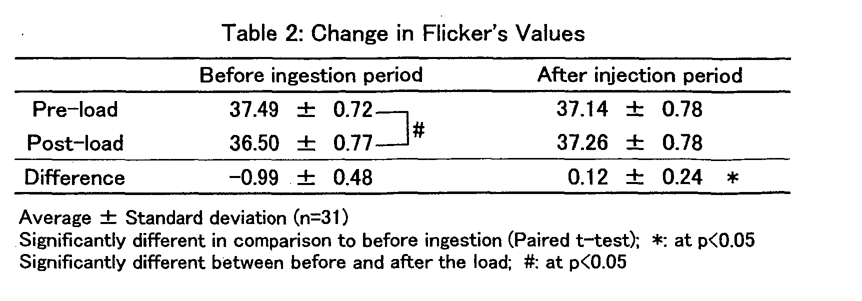

**Table 3: Change in Salivary Cortisol Value**

| | Before ingestion period | After injection period |
|---|---|---|
| Pre-load | 7.69 ± 0.62 | 4.67 ± 0.38 ** |

| | | |
|---|---|---|
| Average ± Standard deviation (n=31) Significantly different in comparison to before ingestion (Paired t-test); **: at p<0.01 | | |

**Table 4: Changes in Work EfFciency and Error Rate in Uchida-Kraepelin's Test**

| | Before ingestion period | After injection period |
|---|---|---|
| Initial half work (entries) | 1066 ± 33 | 1098 ± 34 |
| Latter half work (entries) | 1126 ± 32 | 1142 ± 39 |
| Overall work (entries) | 2192 ± 64 | 2240 ± 72 |
| Increament in the latter half work (%) | 6.0 ± 0.9 | 3.9 ± 1.3 |
| Number of errors (entries) | 4.4 ± 0.5 | 3.2 ± 0.5 * |
| Average rate of errors (%) | 0.20 ± 0.02 | 0.15 ± 0.02 * |

| | | |
|---|---|---|
| Average ± Standard deviation (n=31) Significantly different in comparison to before ingestion (Paired t-test); *: at p<0.05 | | |

As shown in Table 2, although the flicker values are decreased significantly after the calculation load was imposed before the ingestion of dried-bonito extract, a significant difference was not observed for the flicker values between before and after the calculation load after the ingestion. Further, when the values before and after the ingestion of the same extract were compared with regard to the difference in flicker values, it was confirmed that the flicker value difference was increased significantly as the result of the ingestion of the extract. In addition, as shown in Table 3, it was confirmed that the salivary cortisol (stress marker) value prior to the imposition of the calculation loadwas decreased significantly after the ingestion of the dried-bonito extract. Furthermore, as shown in Table 4, a significant difference was not observed in calculation workload between before and after the ingestion of the dried-bonito extract. Meanwhile, with respect to the number of errors and the rate of errors, it was confirmed that the values after the ingestion of the extract were significantly lower than those prior to the ingestion. These results showed that the dried-bonito extract has an anti-stress effect and an anti-fatigue effect with respect to mental load. Further, it was confirmed that the ingestion of the dried-bonito extract causes an increase in efficiency of mental labor and an improvement in learning ability.

### Test Example 4: Confirmation of action for visual fatigue in human

A solution (liquid food composition) of Dried-Bonito Extract A of the present invention that had been prepared in Test Example 1 was provided to a group of 11 males (average age: 45.7 ± 7.8 years old) who perform VDT work for 4 hours or more per day and experience eye strain or dimming , or shoulder stiffness and back stiffness, for ingestion for 28 days in amounts of 125 mL per day. For the purpose of determining the initial values, pre-observation was conducted for a 15-day period just prior to the start of ingestion. Flicker values were measured twice a day (morning and evening) during the ingestion period, and the subjective symptoms were evaluated weekly using a questionnaire format. Thesubjects' eyesight was also measured before and after the ingestion period. Analysis of the flicker values was conducted based on weekly changes in morning and evening values as well as changes in the daily variation 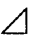 (morning - evening). The results of changes in the subjective symptoms caused by the ingestion of a solution of the dried-bonito extract are shown in Table 5. Table 6 shows the results for measurement of flicker values, while Table 7 shows the results for eyesight measurement. The initial values for flicker value and subjective symptoms are the respective average values for the pre-observation period. In the tables, the values that showed a significant difference at p<0.05 in comparison to the initial values are marked with a * symbol, and the values that showed a trend at p<0.1 are marked with a # symbol.

**Table 5: Changs in Subjective Symptoms**

| | Initial values | 1w | 2w | 3w | 4w |
|---|---|---|---|---|---|
| Eye strain | 3.70 ± 0.88 | 3.36 ± 1.21 | 3.45 ± 1.29 | 3.18 ± 1.25 * | 3.27 ± 1.35 # |
| Eye-ache | 2.79 ± 0.82 | 2.36 ± 0.67 | 2.55 ± 1.04 | 2.73 ± 0.90 | 2.36 ± 0.81 |
| Dimming | 3.48 ± 1.04 | 3.18 ± 1.25 | 3.36 ± 1.21 | 3.18 ± 1.08 | 3.09 ± 1.04 # |
| Tears come into eyes.($1) | 3.42 ± 1.01 | 3.20 ± 1.32 | 3.55 ± 1.13 | 3.09 ± 0.83 # | 3.36 ± 1.12 |
| Eyes become red. | 3.27 ± 1.08 | 2.64 ± 1.03 # | 2.91 ± 1.04 | 2.91 ± 1.22 | 2.64 ± 1.03 * |
| Eyes are dazzled. | 2.79 ± 1.04 | 2.73 ± 1.10 | 2.70 ± 1.16 | 2.91 ± 1.04 | 2.82 ± 0.98 |
| Things see double. | 2.76 ± 0.93 | 2.60 ± 1.07 | 2.73 ± 1.01 | 2.64 ± 1.03 | 2.55 ± 1.04 |
| Stiffness in the shoulders or lower back | 3.55 ± 1.01 | 3.30 ± 1.16 | 3.27 ± 1.35 | 3.36 ± 1.29 | 3.27 ± 1.35 |
| Feel irritated. | 2.70 ± 0.82 | 2.73 ± 1.01 | 2.36 ± 0.67 | 2.73 ± 0.90 | 2.73 ± 0.90 |
| Feel heavyheaded. | 2.58 ± 0.97 | 2.36 ± 0.81 | 2.45 ± 0.93 | 2.55 ± 1.04 | 2.64 ± 1.21 |
| Have a headache. | 2.30 ± 0.90 | 2.27 ± 0.79 | 2.27 ± 0.79 | 2.45 ± 0.93 | 2.55 ± 0.93 |
| Depth of sleep ($2) | 2.79 ± 0.98 | 2.91 ± 1.22 | 2.73 ± 1.01 | 2.82 ± 0.98 | 2.64 ± 0.81 |

| | | | | | |
|---|---|---|---|---|---|
| Average ± Standard deviation (n=11) The smaller the numerical values are, the more the subjective symtoms are improved. $1: The bigger the numerical values are, the less tears come in the eyes. $2: The bigger the numerical values are, the poorer the sleep is. *: at p<0.05, #: at p<0.1 (Eilcoxon test) | | | | | |

**Table 6: Change in Flicker Value**

| | Initial values | 1 w | 2w | 3w | 4w |
|---|---|---|---|---|---|
| Morning | 35.25 ± 2.13 | 35.28 ± 2.17 | 35.39 ± 2.36 | 35.31 ± 2.33 | 35.48 ± 2.23 |
| Evening | 34.23 ± 1.88 | 34.58 ± 2.03 | 34.39 ± 2.13 | 34.61 ± 2.27 | 34.88 ± 1.74 # |
| 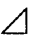 (Morning - Evening) | 1.02 ± 0.87 | 0.69 ± 0.93 | 1.00 ± 0.63 | 0.70 ± 0.97 | 0.60 ± 1.00 |

| | | | | | |
|---|---|---|---|---|---|
| Average ± Standard deviation (n=11) Change in flicker value (morning and evening): It is suggested that the smaller the numerical values are, the more the fatigue is. Change in diurnal variation 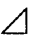 : It is suggested that the bigger the numerical values are, the more the fatigue is. #: at p<0.1 (Paired t-test) | | | | | |

**Table 7: Change in Eyesight**

| | Right | | Left | | Change | |
|---|---|---|---|---|---|---|
| Subject No. | Before the ingestion period | After the ingestion period | Before the ingestion period | After the ingestion period | Right | Left |
| 1 | 1.50 | 1.20 | 0.90 | 0.50 | - | - |
| 2 | 1.20 | 1.20 | 1.50 | 1.50 | No change | No change |
| 3 | 1.00 | 1.00 | 0.60 | 1.20 | No change | + |
| 4 | 1.50 | 1.20 | 1.50 | 1.20 | - | - |
| 5 | 0.30 | 0.50 | 0.15 | 0.20 | + | + |
| 6 | 0.60 | 1.50 | 1.50 | 1.50 | + | No change |
| 7 | 1.00 | 0.60 | 1.00 | 1.00 | - | No change |
| 8 | 1.20 | 1.50 | 1.20 | 1.20 | + | No change |
| 9 | 1.20 | 1.50 | 1.20 | 1.50 | + | + |
| 10 | 1.00 | 1.20 | 1.00 | 1.00 | + | No change |
| 11 | 0.50 | 0.80 | 0.50 | 0.60 | + | + |
| Average Value | 1.00 | 1.11 | 1.00 | 1.04 | | |
| Standard deviation | 0.39 | 0.35 | 0.44 | 0.44 | | |

As shown in Table 5, a downward tendency was observed for the subjective symptom evaluations "eye strain", "eye-ache", "dimming", "tears come into eyes" and "eyes become red" as the result of the ingestion of the aforementioned solution. For "eye strain", in comparison to the initial value of 3.70, the value after 3 weeks of the ingestion had been decreased significantly to 3.18, and a downward tendency (p<0.1) was observed even at the fourth week. A downward tendency (p<0.1) was observed at the fourth week of the ingestion for "eye-ache ", and at the third week of the ingestion for "tears come into eyes" (For "tears come into eyes", the bigger the numerical value are, the less tears come in the eyes, that is, meaning "eye dryness"). For "eyes become red", a downward tendency was observed at the first week of the ingestion, and the value had been decreased significantly at the fourth week.

As shown in Table 6, although the morning values for flicker value were not different, the evening values showed an upward tendency at the fourth week of the ingestion. Diurnal variation 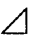 (morning - evening) showed a downward tendency at the third and fourth weeks of the ingestion. The value for 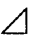 (morning - evening) was 0.60 at the fourth week of the ingestion in comparison to an initial value of 1.02.

As shown in Table 7, for eyesight, an improvement in both eyes or in either the left or right eye was observed for 7/11 subjects (67%), a decrease was observed for 3/11 subjects (27%) and no change was observed for 1/11 subjects (9%).

It is widely known that flicker values are reduced by a VDT load, and that flicker values correlate to the development of mental fatigue or a decrease in the level of arousal and consciousness at the cerebral center, and flicker values are reported to be useful for detecting eye strain (Kakizaki, T. et al., Changes in mental workload and fatigue during performance of a mental task, Japanese Journal of Industrial Health, 34: 565-573, 1992 (an article in Japanese)). It is also reported that decreased retina function plays an important role in decreasing flicker values (Iwasaki, T. and Kurimoto, S., Changes of colored critical flicker fusion values during the experimental repetitive task with display screen , Journal of Japanese Ophthalmological Society, 91(3): 65-71, 1987 (an article in Japanese)). Regarding "tears come into eyes", it is said that most patients who complain of eye strain have dry eyes, and that eye dryness is closely related to visual fatigue. The term "dimming " is sometimes used synonymously with weakened eye focus (the report of Kakizaki cited above), and considering the average age of 45.7 years, the possibility of symptoms caused by accommodative insufficiency caused by spectacles for the aged can be presumed. The cause thereof is a decline in the function of ciliary muscle, and it is considered that a complaint that "eyes are tired" occurs as a result of this. The above results indicate the possibility that the central nervous system of the whole body including nerves of retina and ciliary body is improved by the ingestion of dried-bonito extract.

### Test Example 5: Confirmation of action for model mice under restraint stress

Distilled water or Bonito Extract A that was prepared in Test Example 1 (solution with a solid content of 4. 0 %, including 3.0 % sodium chloride) was orally administered using a sonde to 2 groups (control group and test group) of 6 mice (each group n = 6) consisting of five-week old CDF-1 male mice, respectively, in an amount representing 20 mL per 1 kg of body weight. After 1 hour of administration, the mice were inserted into vinyl chloride tubes having an internal diameter of 2.5 cm and a length of 10 cm, and imposed with a restraint load for 40 to 60 minutes. Thereafter, the amount or activity of corticosterone, lactate dehydrogenase (hereunder, referred to as "LDH") and glutamate/oxaloacetate transaminase (hereunder, referred to as "GOT") that are reported as being stress markers in blood was measured according to an ordinary method.

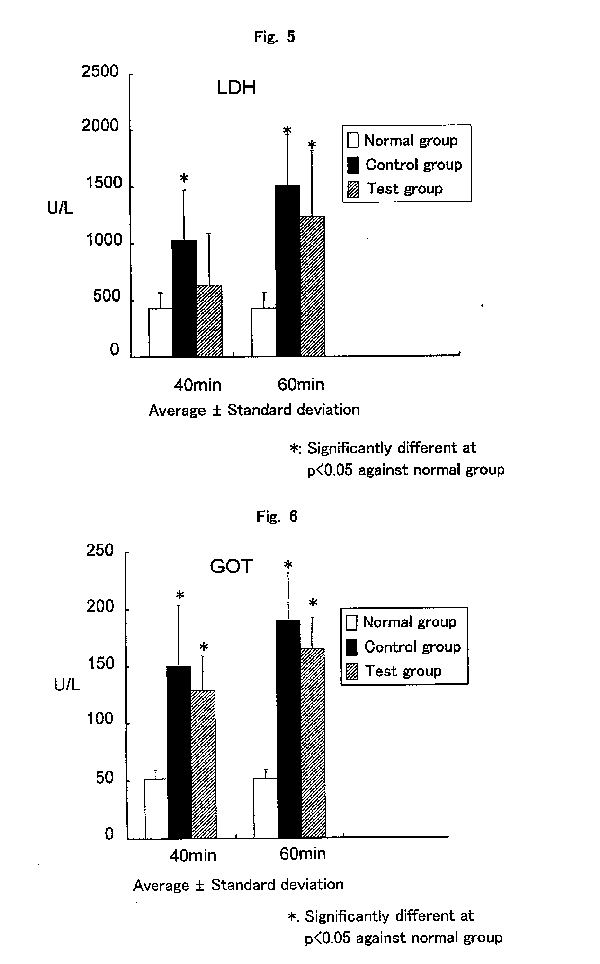

The results are shown in the below-presented Figure 4, Figure 5 and Figure 6. At this time, the same measurement was also performed for mice on which a restraint stress load was not imposed (n=6, indicated by "Normal group" in the figures) . As shown in the figures, for the control group, corticosterone amount (Figure 4), LDH activity (Figure 5) and GOT activity (Figure 6) were all increased due to restraint stress. In contrast, for the group administered with the Bonito Extract A, each of these values was low in comparison to the control group. That is, it was observed that the stress markers of the model mice under stress was reduced by administration of the bonito extract. From the above results also, it was confirmed that a bonito extract has a stress alleviating action.

### Example 1: Preparation of packaged liquid food from dried-bonito extract (liquid food composition)

After crushing 250 kg of a dried bonito (katsuo ara-hombushi in Japanese) that had been prepared by an ordinary method, the crushed mass was immersed into 1,000 kg of ion exchanged water that had been heated to 95 °C, after which extraction was conducted at 90 °C for 45 minutes, followed by cloth filtering and membrane filtering to obtain an extract (solid content: 5.0 %). The resulting extract was subjected to a nitrogen purge and then sterilized by heating at 130 °C for 60 seconds. Thereafter, 100 mL of the extract was packed in a paper pack container having an interior that had been coated with aluminium. The dissolved oxygen concentration at this time was 1. 2 ppm. The solid content of the dried-bonito extract within the container was 5.0 g.

### Example 2: Preparation of bonito extract powder (powdered food composition)

After adding dextrin (Sanwa Cornstarch Co. , Ltd., "Sandec #100") to the liquid of Bonito Extract A (solid content: 30 %) that had been prepared in Test Example 1, in an amount equivalent to the solid content of the extract, and dissolving, the resulting solution was dried by spray drying to obtain a bonito extract powder. 3 G of this powder was packed in an aluminum pouch bag to obtain a packaged product containing 1. 5 g of the solid content of the bonito extract per one meal (1 bag). In this connection, for an extract that had been prepared without adding diatomaceous earth to the raw material bonito broth used in Test Example 1, conducting cloth filtration and membrane filtration, and then desalting using an electrodialyzer (Acilyzer, manufactured by Asahi Kasei Corporation), dextrin was added in an amount equivalent to the solid content of the extract in the same manner as described above and spray drying was performed to obtain a bonito extract powder as a comparison control. 2 % hot water solution was prepared for the bonito extract powder of the present invention and the bonito extract powder as the comparison control, and sensory evaluation was conducted by a taste evaluation panel consisting of 20 persons. The results are shown in Table 8. As shown in Table 8, it was shown that, as compared to the bonito extract as the comparison control, a fishy smell was reduced in the bonito extract of the present invention, and the taste and flavor thereof were preferable. Based on this result, it was confirmed that a food composition having an anti-stress action that is easier to drink can be provided according to the present invention.

**Table 8: Results of Sensory Test**

| | Inventive Extract | Control Extract |
|---|---|---|
| Strength of fishy smell | 2 | 18* |
| Strength of strange taste | 1 | 19* |
| Preferableness of taste | 17* | 3 |
| Preferableness of flavor | 18* | 2 |
| Preferableness of aroma | 18* | 2 |
| Comprehensive preferableness | 18* | 2 |

| | | |
|---|---|---|
| Numerals indicate the number of the persons who chose the strength or preferableness. Significantly different at p<0.05. | | |

### Example 3: Preparation of dried-bonito extract powder (powdered food composition)

After adding dextrin(Sanwa Dempun Kogyo Kabushiki Kaisha, "Sandec #100") to a liquid of Dried-Bonito Extract A (solid content: 5 %) that had been prepared in Test Example 1, in an amount equivalent to the solid content of the extract, and dissolving, the resulting mixture was dried by lyophilization to obtain a dried-bonito extract powder. 3 G of this powder was packed in an aluminum pouch bag to obtain a packaged product containing 1. 5 g of the solid content of the dried-bonito extract per one meal (1 bag).

### Example 4: Preparation of granules of dried-bonito extract (granular food composition)

100 Parts by weight of the dried-bonito extract powder prepared in Example 3 was added and mixed with 0.5 parts by weight of aspartame (manufactured by Ajinomoto Co., Inc.), 10 parts by weight of sodium glutamate ("MSG-RC", manufactured by Ajinomoto Co., Inc.) and 3. 5 parts by weight of tap water. The resulting mixture was then subjected to granulation, using an extrusion granulating machine, to obtain granules of the dried-bonito extract. 3 G of these granules was packed in an aluminum pouch bag to obtain a packaged product containing 1.43 g of the solid content of the dried-bonito extract per one meal (1 bag).

### Example 5 : Preparation of tablets of dried-bonito extract (food composition in tablet form)

100 Parts by weight of the dried-bonito extract powder prepared in Example 3 was added and mixed with 0.5 parts by weight of aspartame (manufactured by Ajinomoto Co., Inc.) and 5 parts by weight of sodium glutamate ("MSG-FC", manufactured by Ajinomoto Co., Inc.). The resulting mixture was subjected to tabletting, using a tabletting machine, to obtain tablets having a weight of 2.2 g/tablet and comprising the dried-bonito extract. The weight of the solid material of the dried-bonito extract for each of these tablets was 1.05 g.

### Example 6: Preparation of capsules of dried-bonito extract (capsular food composition)

100 Parts by weight of the dried-bonito extract powder prepared in Example 3 was packed in gelatin-made No. 0 hard capsules (manufactured by Japan Aliment Industry Co., Ltd.) in an amount of 400 mg per 1 capsule, to obtain capsular product containing the dried-bonito extract powder. The weight of the solid content of the dried bonito extract contained in 5 of these capsules (ingestion amount per one meal) was 1.0 g.

### Example 7: Preparation of jelly food of dried-bonito extract (jelly food composition)

4.0 Parts by weight of gelatin (manufactured by Nitta Gelatin Inc.) was added to 100 parts by weight of the liquid Dried-Bonito Extract A obtained in Test Example 1, 100 parts by weight of water, 0.2 parts by weight of aspartame (manufactured by Ajinomoto Co., Inc.) and 2.0 parts by weight of sodium glutamate. The resulting mixture was dissolved by heating, 100 g of the solution was packed into an aluminium container, sterilization was conducted for 15 minutes at 120 °C under pressure, and the solution was then cooled to obtain a dried-bonito extract food in a jelly form. The weight of the solid content of the dried-bonito extract contained per one meal of this food composition was 2.42 g.

### Example 8: Preparation of Japanese clear soup-like food composition (liquid food composition)

10 Kg of the liquid Dried-Bonito Extract A obtained in Test Example 1 (before sterilization), 6 kg of heavy soy sauce (Koi-kuchi shoyu in Japanese), and 0.1 kg of sodium glutamate ("MSG-RC", manufactured by Ajinomoto Co., Inc.) were mixed to dissolution. The liquid thus obtained in this manner was subjected to a nitrogen purge after being sterilized by heating at 120 °C for 60 seconds, and then asepticallypackaged in aluminum pouches in an amount of 40 g in each pouch, to obtain a packaged liquid food of the present invention. The weight of the solid content of the dried-bonito extract per one meal (1 bag) of the obtained food was 1.24 g. The dissolved oxygen concentration of this liquid composition was 0.9 ppm.

### Example 9: Preparation of powdered miso soup-like food composition (powdered food composition)

6.0 Kg of the dried-bonito extract powder obtained in Example 3 and 7.5 kg of powdered miso (manufactured by Hanamaruki Co., Ltd.) were mixed to obtain a powdered miso soup-like food powder. The powder was packed in aluminum pouches in an amount of 13.5 g in each pouch, to obtain a powdered miso soup-like packaged food. The weight of the solid content of the dried-bonito extract contained per one meal of this food composition was 3.0 g.

### Example 10: Preparation of powdered noodle soup-like food composition (powdered food composition)

1.0 Kg of the dried-bonito extract powder obtained in Example 3, 2.4 kg of powdered soy sauce (manufactured by Yamasa Corporation), 0.7 kg of sodium chloride, 0.3 kg of granulated sugar and 0.2 kg of powdered rice wine were crushed and mixed to obtain a powdered broth-like food. The thus-obtained powder was packed in aluminum pouch bags in an amount of 23 g in each bag, to obtain a packaged product of the powder broth-like food composition. The weight of the solid content of the dried-bonito extract contained per one meal (1 bag) of this composition was 2.5 g.

### Industrial Applicability

According to the present invention, there can be easily provided a food composition having an anti-stress action, an anti-fatigue action with respect to mental load, and an action that enhances the efficiency of mental labor as the results of these actions, which food composition can prevent or alleviate fatigue such as eye strain, or the like, mental fatigue or mental dysfunction that are felt on a daily basis or a state deriving from stress such as an unstable mental state.

## Claims

1. A food composition having an anti-stress action, which composition comprises, as an active ingredient, an extract of migratory fish.

2. The food composition having an anti-stress action as set forth in Claim 1, which further comprises, as an additive, at least one member selected from the group consisting of other nutritional components, other anti-fatigue components, taste-improving components, excipient components, bacteriostatic components and pigments, in addition to said extract of migratory fish.

3. The food composition having an anti-stress action as set forth in Claim 1 or 2, wherein said migratory fish is of Perciformes Scombrina.

4. The food composition having an anti-stress action as set forth in Claim 1 or 2, wherein said migratory fish is bonito.

5. The food composition having an anti-stress action according to any one of Claims 1 to 4, wherein said extract of migratory fish is an extract that has been extracted from bonito meat.

6. The food composition having an anti-stress action according to any one of Claims 1 to 4, wherein said extract of migratory fish is an extract that has been obtained by enzymatic hydrolysis of a broth of bonito meat.

7. The food composition having an anti-stress action according to any one of Claims 1 to 4, wherein said extract of migratory fish is an extract that has been obtained by heat-concentrating a broth and/or a steam-cooked broth of bonito meat, and adding thereto diatomaceous earth of an amount of 2-20 % relative to the extract solid content, followed by filtration.

8. The food composition having an anti-stress action according to any one of Claims 1 to 4, wherein said extract of migratory fish is an extract that has been obtained by subjecting dried fish made in turn from migratory fish to hot-water extraction.

9. The food composition having an anti-stress action according to any one of Claims 1 to 4, wherein said extract of migratory fish is an extract that has been obtained by subjecting dried fish made from migratory fish to enzymatic hydrolysis with a protease, followed by subjecting the thus-treated mass to hot-water extraction.

10. The food composition according to any one of Claims 5 to 9, wherein said extract of migratory fish is a permeated liquid that has been obtained by treating an extract obtained according to the method of any one of Claims 5 to 9 with an ultrafiltration membrane having a nominal molecular cutoff of 500 to 5,000.

11. The food composition according to any one of Claims 5 to 9, wherein said extract of migratory fish is a permeated liquid that has been obtained by treating an extract obtained according to the method of any one of Claims 5 to 9 with a reverse osmosis membrane having a nominal salt rejection of 10 % or less.

12. A food composition having an anti-stress action, comprising, as an effective ingredient, a mixture of nutritional components consisting of (a) a total of 294 mg or more of nitrogen-containing compounds such as peptides, proteins, amino acids, and the like, derivable from a bonito extract, (b) a total of 55 mg or more of organic acids consisting of lactic acid, citric acid, malic acid and succinic acid; (c) 1 mg or more of nucleic acid-related compounds; and (d) a total of 16 mg or more of mineral components such as NaCl and KCl, at a ratio by weight of, when the amino acid amount is defined as 1, from 0.5 to 2 of the organic acid, from 0.001 to 0.5 of the nucleic acid-related compound and from 0.02 to 2 of the mineral component.

13. The food composition having an anti-stress action according to any one of Claims 1 to 12, which food composition contains 0.5 g or more of an extract of migratory fish per one meal in terms of the solid content thereof.

14. The liquid food composition having an anti-stress action according to any one of Claims 1 to 13, wherein said liquid food composition has been prepared by packaging aseptically a liquid extract of migratory fish in such that it would contain 0.5 g or more per one meal of the extract of migratory fish in terms of the solid content thereof, and such that the dissolved oxygen concentration in the liquid extract is 5 ppm or less (25 °C) .

15. The powdered food composition having an anti-stress action according to anyone of Claims 1 to 13, wherein saidpowdered food composition has been prepared by adding excipient components and taste-improving components to an extract of migratory fish in such that the resulting mixture would contain 0.5 g or more per one meal of the extract of migratory fish in terms of the solid content thereof, followed by powdering the mixture.

16. The granular food composition having an anti-stress action according to anyone of Claims 1 to 13, wherein saidgranular food composition has been prepared by adding excipient components and taste-improving components to an extract of migratory fish in such that the resulting mixture would contain 0.5 g or more per one meal of the extract of migratory fish in terms of the solid content thereof, followed by granulating the same.

17. The tablet-type food composition having an anti-stress action according to any one of Claims 1 to 13, wherein said tablet-type food composition has been prepared by adding excipient components and taste-improving components to an extract of migratory fish in such that the resulting mixture would contain 0.5 g or more per one meal of the extract of migratory fish in terms of the solid content thereof, followed by tabletting the same.

18. The capsular food composition having an anti-stress action according to anyone of Claims 1 to 13, wherein said capsular food composition has been prepared by adding excipient components to an extract of migratory fish in such that the resulting mixture would contain 0.5 g or more per one meal of the extract of migratory fish in terms of the solid content thereof, followed by powdering the mixture, and packing the powder in capsules.

19. The capsular food composition having an anti-stress action according to anyone of Claims 1 to 13, wherein said capsular food composition has been prepared by concentrating an extract of migratory fish in such that the resulting paste would contain 0.5 g or more per one meal of the extract of migratory fish in terms of the solid content thereof, followed by packing the paste in capsules.

20. The jelly food composition having an anti-stress action according to any one of Claims 1 to 13, wherein said jelly food composition has been obtained by adding at least one kind of gelling agent selected from the group consisting of agar, gelatin, starches and gums to an extract of migratory fish in such that the resulting mixture would contain 0.5 g or more per one meal of the extract of migratory fish in terms of the solid content thereof.

21. The food composition according to any one of Claims 1 to 20, wherein said anti-stress action is for improving learning ability.

22. The food composition according to any one of Claims 1 to 20, wherein said anti-stress action is for improving asthenopia.
